# EUROPEAN PATENT SPECIFICATION

(11) **EP 2 674 155 B1**
(45) Date of publication and mention of the grant of the patent: **02.08.2017**
(21) Application number: 13171794.4
(22) Date of filing: 13.06.2013
(51) Int. Cl.: A61K 31/05, A61K 31/7024, A61P 29/00, A61P 31/12, A61P 3/00, A61P 9/00

(54) **Association of resveratrol and carboxymethylglucan**
Assoziation von Resveratrol und Carboxymethylglucan
Association de resvératrol et carboxyméthylglucan

(30) Priority: 13.06.2012 IT RM20120275
(43) Date of publication of application: 18.12.2013
(73) Proprietor: Noos S.r.l., 00181 Roma (IT)
(72) Inventor: Moretti, Giancarlo, 00181 Rome (IT); Mosca, Luciana, 00178 Rome (IT)
(74) Representative: Germinario, Claudio

(56) References cited:
- WO-A1-2005/012507
- WO-A1-2008/010241
- WO-A1-2009/012551
- A. AMRI ET AL: "Administration of resveratrol: What formulation solutions to bioavailability limitations?", JOURNAL OF CONTROLLED RELEASE, vol. 158, no. 2, 1 March 2012 (2012-03-01) , pages 182-193, XP055051019, ISSN: 0168-3659, DOI: 10.1016/j.jconrel.2011.09.083

## Description

The present invention relates to the association of resveratrol and carboxymethylglucan (CMG) in order to increase solubility in aqueous solution and consequent bioavailability of resveratrol, as well as to strengthen the stability of this molecule. The present invention further relates to pharmaceutically compositions, dietary supplies, medical devices, cosmetics, functional pieces of food and veterinary products and the use thereof.

### State of art

Resveratrol (3,4',5-trihydroxystilbene) belongs to a class of polyphenols defined stilbenes or phytoalexins and it is produced by the plants in response to stresses, damages, infections caused by fungi and ultra-violet (UV) rays. It can be found in high concentrations in the peels of red grape, as well as in raspberries, mulberries, bilberries and in *Polygonum cuspidatum,* a perennial herbaceous plant belonging to the same family of buckwheat, known as from ancient times as laxative herb and occasionally used as food. The extracts of *P*. *cuspidatum* represent a rich source of resveratrol, as the concentration of this polyphenol in the extract can reach even 98%. During the last years it has been demonstrated that this compound has several advantageous effects with respect to neurodegenerative pathologies, tumours and atherosclerosis. The antioxidant properties and the ability of modulating some of the most important intracellular signalling routes (mainly those mediated by NF-kB) are the main elements at the basis of its activity (1-3). Thanks to the antioxidant action thereof, resveratrol could be able to interfere with the intracellular redox status involved in the viral infections. In fact, it has demonstrated *in vitro* a strong antiviral activity against a wide number of viruses, thereamong the one of A influence, viruses herpes simplex 1 and 2, cytomegalovirus, varicella-zoster virus, orthomyxovirus, adenovirus, respiratory syncytial viruses and polyomavirus (4,5). Moreover, it seems having a synergic effect with antiviral drugs used against HIV virus (6,7). Notwithstanding the mentioned very interesting properties, resveratrol is still used in limited way in pharmaceutically formulations substantially for two reasons: poor solubility in aqueous solution and limited stability. In fact, resveratrol is a lipophilic polyphenol and, as such, insoluble in itself in aqueous environments (solubility in water -0.03 mg/ml (8)). This makes difficult the use thereof in water-based liquid products and it is also a problem for the bioavailability of the molecule once ingested. Apart from this, resveratrol is not stable, but tends to be subjected to oxidizing reactions altering the properties thereof. To confirm this, currently there are several resveratrol-based products on the market, most part under the form of tablets, capsules or powder. Therefore it was felt in the state of art the need of increasing the solubility in aqueous solution of resveratrol and the stability thereof.

A. AMRI ET AL: "Administration of resveratrol: What formulation solutions to bioavailability limitations?", JOURNAL OF CONTROLLED RELEASE, vol. 158, no. 2, 1 March 2012, pages 182-193 discloses an improvement of bioavailability of resveratrol with zink-pectine beads, modified with glutaraldehyde or chitosane addition. Surprisingly it has been found that an association of resveratrol and CMG (carboxymethylglucan) succeeds in increasing the solubility of resveratrol and even the stability thereof.

A first object of the present invention is an association of resveratrol and/or salts thereof and CMG. In an embodiment such association is constituted by resveratrol and CMG.

A second object of the present invention is an association of resveratrol and/or salts thereof and CMG for use in a human or veterinary therapeutic treatment in particular of inflammatory, viral, diabetic, cardiac, neurodegenerative, atherosclerotic pathologies. In an embodiment such association is constituted by resveratrol and CMG.

Another object of the present invention is a pharmaceutical composition comprising, as active principle, an association of resveratrol and/or pharmaceutically acceptable salts thereof and CMG and one or one or more pharmaceutically acceptable vehicles and/or diluents and/or excipients, for use in a human or veterinary therapeutic treatment, in particular of inflammatory, viral, diabetic, cardiac, neurodegenerative, atherosclerotic pathologies. In an embodiment such composition comprises, as active principle, an association constituted by resveratrol and CMG. Additional object of the present invention are a medical device and/or a dietary supply and/or a functional food and/or a cosmetic product comprising an association of resveratrol and/or salts thereof and CMG and one or more pharmaceutically acceptable vehiculants and/or diluents and/or excipients for human and veterinary use.

Additional object of the present invention is a process for preparing the composition according to the present invention, wherein the association comprising resveratrol and CMG is formulated into suitable dosage units with one or more pharmaceutically acceptable vehiculants and/or diluents and/or excipients.

Nine figures are enclosed to the present invention, wherein:

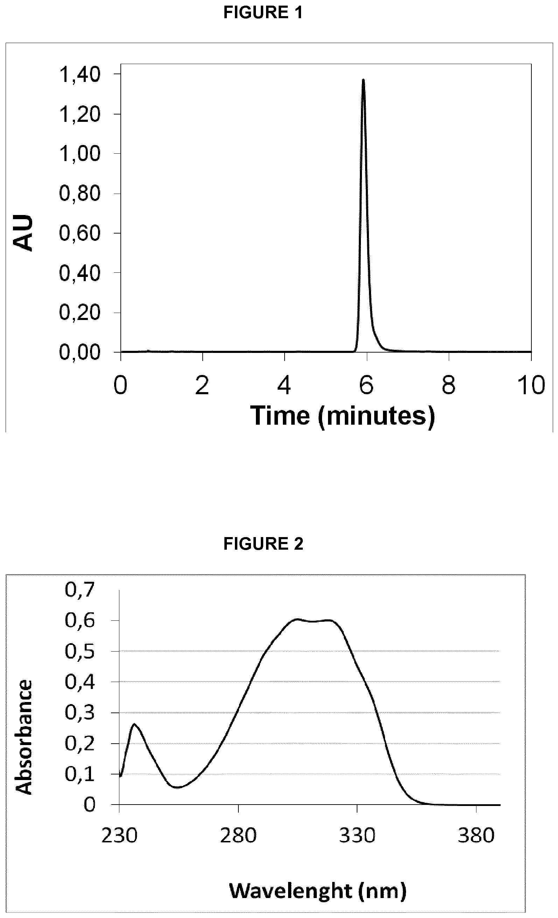

**figure 1** shows the chromatogram of a sample containing resveratrol, the retention time thereof results to be about 6 min, under the chromatographic conditions described below;
**figure 2** shows the UV-Vis absorption spectrum of resveratrol of the peak shown in figure 1, with the one maximum typical at 306 nm;
**figure 3** shows the percentage of resveratrol with respect to the expected one detected in HPLC for aqueous solutions containing increasing concentrations of resveratrol and CMG in ratio 1:1 compared to a solution of sole resveratrol;
**figure 4** shows the percentage of resveratrol with respect to the expected one detected in HPLC for aqueous solutions containing constant concentrations of resveratrol (0.5mg/ml) and increasing concentrations of CMG, compared to a solution of sole resveratrol;
**figure 5** shows the stability in time of resveratrol in aqueous solution a 0.03 mg/ml (solubility limit in aqueous solution);
**figure 6** shows the stability in time of resveratrol (0.5 mg/ml) in aqueous solution in presence of CMG 1 mg/ml;
**figure 7** shows the stability in time of resveratrol (1 mg/ml) in aqueous solution in presence of CMG (1 mg/ml);
**figure 8** shows the metabolic activity of VERO cells in presence of resveratrol 0.5 mg/ml, CMG 1 mg/ml or association of the two molecules, 6 hours after exposition;
**figure 9** shows the increase in solubility of acetyl-resveratrol and trimethyl ether of resveratrol in presence of CMG 0.1 %, respectively, compared to the solubility value of the pure compound taken as reference.

### Description of the invention

The present invention relates to an association of resveratrol and/or pharmaceutically acceptable salts thereof and CMG wherein the two components are present in a weight ratio of 20:1 up to 1:100.

In a preferred embodiment, the two components of the association are used preferably in the following concentration ranges even if inside the weight ratio mentioned above:
Resveratrol: solution from 0.003% to 10% in weight in aqueous solution
CMG: solution from 0.003% to 10% in weight in aqueous solution

The doses of the products deriving from the present invention could vary according to the pharmacodynamic features of a specific agent, the administration modes and route, race, age, sex, weight, state of health of user, nature and extension of the symptom, type of concurring treatment, frequency of treatment.

CMG is a compound having significant bioprotective properties, as antimutagenic, antigenotoxic, antioxidant and antitumural agent (9-11). It is thought that the mechanism which is at the basis of the protective effects of CMG is the capability thereof of acting as "scavenger" of the reactive species of oxygen (12), already at very low concentrations. Moreover, CMG has demonstrated to increase significantly the levels of T-lymphocytes CD4+ and CD8+ in patients with cancer of the prostate (13) and to stimulate the production of TNF-alfa by the tissue macrophage by increasing the phagocytic activity thereof (14,15). These data underline then for this molecule considerable immunomodulating properties.

CMG, in the scope of the present patent, relates to the product as such, of any origin.

Resveratrol, in the scope of the present patent, is meant in the available forms: product extracted from *P*. *cuspidatum* or from other vegetable sources, various forms of the molecule available on the market or synthetized in laboratory, all possible salts of resveratrol obtained by means of salifications, substitutions, derivatisations and/or bonds to reactive groups of the molecule. As salts suitable in the scope of the present invention those compatible with the pharmaceutical, nutraceutical and cosmetic formulations can be mentioned. Among the vehiculants and/or acceptable diluents and/or excipients, according to the administration route and the practice of the pharmaceutical/nutritional standards the following one can be mentioned:
- for the liquid formulations preferably a vehicle comprising: sucrose or sucrose with glycerin and/or mannitol and/or sorbitol, polysorbate 20, PEG, propylene glycol;
- for the formulations under suspension and emulsion preferably a vehicle comprising: a natural rubber, agar, sodium alginate, pectine, methylcellulose, carboxymethylcellulose or polyvinyl alcohol.
- for the injectable formulations preferably a vehicle comprising: sterile water, olive oil, ethyl oleate, propylene glycol and an adequate quantity of hydrochloride lidocaine;
- for the solid formulations preferably a vehicle comprising one or more diluents such as: lactose, dextrose, sucrose, cellulose, corn starch; one or more lubricants such as: silica, stearic acid, magnesium or calcium stearate, polyethylene glycols; one or more binding agents (agglutinants) such as: starches, mucilages, gelatine, methycellulose, carboxymethylcellulose, or polyvinylpyrrolidone; one or more disaggregating agents such as: starch, alginic acid, alginates or glycolates; effervescent mixtures; desiccants; sweeteners; one or more humectant agents such as: lecithin, polysorbate, lauryl sulphate; known substances, inactive and not toxic from the pharmaceutical/nutraceutic point of view.

The vehiculants, diluents and excipients mentioned above are meant exclusively by way of example, however not constituting an exhaustive list.

The advantages of the association according to the present invention are the following ones:
1) the solubilization of resveratrol and the stabilization thereof in aqueous solution;
2) the greater stability of polyphenol in solution constituted an advantage from the industrial point of view for the production of liquid formulations. In fact, this association allows obtaining a prolonged shelf-life of resveratrol, since the molecule is not subjected to degradation or at least the degradation takes place with delayed mode;
3) at productive level, the solubilization of resveratrol allows using a lower quantity of this molecule in the production of liquid formulations of pharmaceutical specialties, medical devices, dietary supplies, cosmetic products, functional pieces of food, veterinary products;
4) the greater solubility and stability of resveratrol allows a better bioavailability thereof. This makes that at the tissue level higher concentrations of this molecule are reached in presence of CMG with respect to the ingestion of sole resveratrol, with consequent increase in efficiency;
5) the solubilization of resveratrol allows the use thereof even in formulations for parenteral, enteral and/or intrathecal use;
6) given that CMG solubilizes resveratrol by making it more bioavailable and consequently more effective, it strengthens even the possible synergetic effects already known between resveratrol and other molecules.

Resveratrol solubility and stability data will be shown hereinafter, obtained by using an association wherein the concentrations of resveratrol and CMG vary in an aqueous solution.

### Methods

### Analysis method in HPLC

The chromatographic method devised for determining resveratrol provides the use of a HPLC Waters system constituted by a pump, 600 model, by a Waters self-sampler mod. 717 and by a UV-Visible Photodiode array detector mod 2996. The system is handled by the Millenium³² software. The used chromatographic column is a Symmetry C-18 with length of 15 cm, associated to a specific pre-column of same material with length of 1 cm. The elution is performed under isocratic conditions by using the following mixtures:
1) 30% [Acetic acid 0.1 % in methanol] e 70% [Acetic acid 0.1 % in water]
2) 80% [Acetic acid 10% in water] and 20% [Acetonitrile].
For the measurements 50 µL of sample are taken, diluted in 500µL of mobile phase and an aliquot of 50µL is injected in HPLC.

### Method for evaluating the cellular metabolic activity

The cellular metabolic activity is evaluated on VERO cellular line, by means of MTT cellular proliferation assay (3-(4,5-dimethylthiazole-2-il)-2,5-diphenyltetrazolium bromide). The test with MTT is a simple method for determining the metabolic activation of the cells and it is based upon the action of the mitochondrial enzyme succinate dehydrogenase reducing the tetrazolium salts in crystals of a bluish product called formazan. Therefore the reaction can take place only in the metabolically active cells and the value of optical density (O.D.), obtained by means of spectrophotometric reading, can be correlated to the quantity of existing vital cells.

### Preparation of the solutions

The solutions of CMG and resveratrol have always been prepared in a buffered, isotonic, physiological pH-containing aqueous solution, constituted by:
- dipotassium phosphate 0.74%
- sodium chloride 0.42%
- potassium phosphate 0.175%

All solutions were cold-prepared and without using any organic solvent.

### Determining the solubility of resveratrol

In order to evaluate the solubility, with consequent effect on the bioavailability thereof, formulations were prepared containing resveratrol in association with CMG at increasing concentrations (from 0.5 mg/ml to 10 mg/ml) in a ratio 1:1, in an aqueous solution of the above-mentioned composition. Starting as from the obtained results additional tests were performed by keeping constant the concentration of resveratrol at 0.5 mg/ml and by increasing the concentration of CMG from 0.025 mg/ml to 4 mg/ml. The results show a high solubilizing activity of CMG on resveratrol.

### Determining the stability of resveratrol

Resveratrol in aqueous environment is not stable, but it tends to be subjected to degradation reactions.

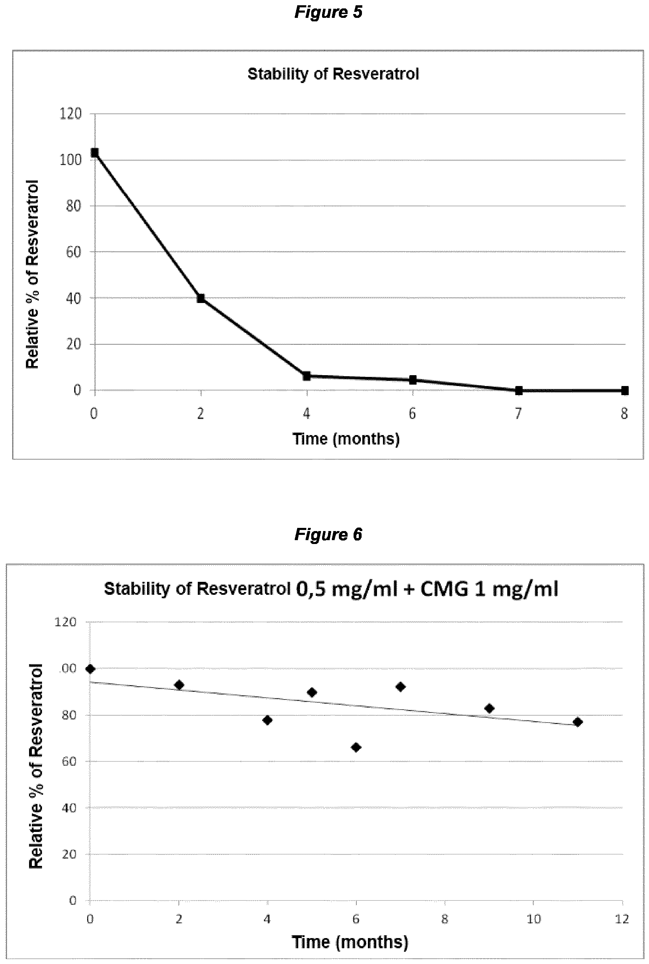

Figure 5 shows the data related to the concentration of resveratrol detected in a buffer solution after 8 months of preservation at controlled temperature (25°C), in closed environment and protected from light. The examined concentration of the solution of resveratrol was 0.03 mg/ml, corresponding to the maximum quantity which can be solubilized in water of the original product. It is noted that already after 2 months a strong degradation of the product takes place, which is no more detected after 7 months. In order to evaluate the stabilizing effect of CMG on the molecule of resveratrol in aqueous solution 2 solutions were prepared by keeping fixed the concentration of CMG (1 mg/ml) and by varying that of resveratrol (0.5 mg/ml and 1 mg/ml), with ratio CMG:resveratrol respectively of 2:1 and 1:1. The two solutions were kept under the same conditions described above for the solution of resveratrol only and they were evaluated in different moments as from the time of the preparation thereof. It was found that CMG is able to stabilize the polyphenol in in aqueous solution, by avoiding the degradation thereof.

### Determining of the cellular metabolic activity

Upon considering the application of the association CMG-resveratrol for the formulation of pharmaceutical products, dietary supplies, medical devices, cosmetic products, functional pieces of food, veterinary products, with consequent interaction with the biological systems of men and animals in general, even the possible cytotoxycity was evaluated. In particular tests of cellular metabolic activity in presence of resveratrol and CMG on VERO cellular line were performed. The cells were sowed on 96-well plates at a density equal to 7000 cells per well. 24 hours after sowing, the culture medium was replaced by means containing resveratrol and/or CMG at the following concentrations:
- Resveratrol 0.5 mg/ml
- CMG 1 mg/ml
- Resveratrol 0.5 mg/ml + CMG 1 mg/ml

6 hours after exposition to these substances MTT colouring agent was added, then the formazan produced by the metabolically active cells was extracted with isopropanol and the optical density measured at 570 nm by subtracting the reference value at 690 nm.

The obtained results show that in presence of CMG the effect of reducing the metabolic activity exerted by resveratrol when it is used on cellular cultures at so high concentrations is limited.

The products object of the present invention can be used for preparing pharmaceutical compositions, dietary supplies, functional pieces of food and medical devices, with antioxidant, antinflammatory, antiviral, antidiabetic, cardioprotective, neuroprotective and chemioprotective properties.

The products object of the present invention can be used for preparing cosmetic products, with anti-aging and antioxidant properties.

The present invention can be applied in the formulation of the pharmaceutical compounds, dietary supplies, medical devices, cosmetic products, functional pieces of food and products for veterinary use. The association, the pharmaceutical and veterinary compositions, the dietary supplies, the medical devices, the functional pieces of food and the cosmetic products according to the invention can be used in the oral, topic, parenteral, enteral, intratecal use. As administration forms, liquid or gel forms can be mentioned constituted by aqueous solutions, syrups, emulsions, ointments, preparations for infusion, for intraperitoneal, subcutaneous, intramuscular administration or solid forms such as tablets, capsules, powder, granulate, as this association improves the bioavailability of resveratrol. Advantageously, the association according to the invention can be associated to other pharmaceutically/nutraceutically active molecules. Some illustrating, not limiting, examples of the invention are shown hereinafter.

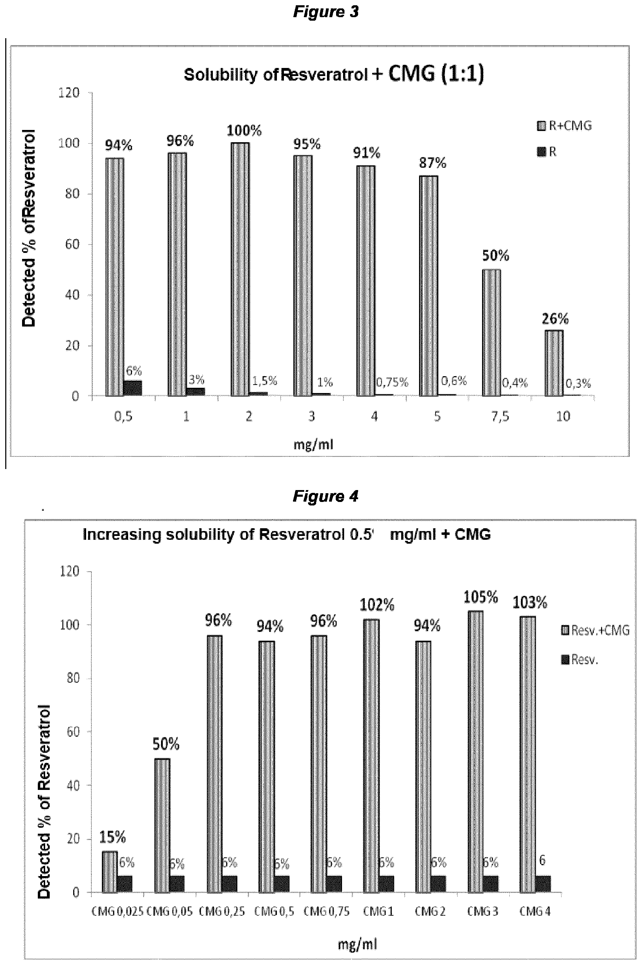

### Example 1 (Figure 3)

In order to evaluate the solubility of resveratrol, with consequent effect on the bioavailability thereof, formulations containing resveratrol in association with CMG at increasing concentrations (0.5 mg/ml to 10 mg/ml) in ratio 1:1 were prepared. Resveratrol and CMG were solubilized in aqueous solution constituted by:
- dipotassium phosphate 0.74%
- sodium chloride 0.42%
- potassium phosphate 0.175%

The tested concentrations of CMG and resveratrol were the following ones:
- CMG 0.5 mg/ml + resveratrol 0.5 mg/ml
- CMG 1 mg/ml + resveratrol 1 mg/ml
- CMG 2 mg/ml + resveratrol 2 mg/ml
- CMG 3 mg/ml + resveratrol 3 mg/ml
- CMG 4 mg/ml + resveratrol 4 mg/ml
- CMG 5 mg/ml + resveratrol 5 mg/ml
- CMG 7,5 mg/ml + resveratrol 7.5 mg/ml
- CMG 10 mg/ml + resveratrol 10 mg/ml

The determination of resveratrol in solution was performed in HPLC according to the method described in the specific section.

From the obtained data (Figure 3) an optimum solubilization of resveratrol is noted, perceptible even with a simple viewing examination, with a solubilization at least 100 times higher than what reported in literature for this polyphenol the limit of solubility in water thereof is 0.03 mg/ml. At concentrations higher than 5 mg/ml a saturation effect is noted, that is the solubilized concentration of resveratrol remains constant and it is no more compared to the existing quantity of CMG.

### Example 2 (Figure 4)

The solubility of resveratrol in an aqueous solution (of the same composition described in example 1) in presence of CMG was evaluated, by using solutions wherein the concentration of resveratrol was kept constant at 0.5mg/ml, whereas the concentration of CMG varied. In particular the following concentrations of CMG were prepared and tested: 0.025mg/ml, 0.05 mg/ml, 0.25 mg/ml, 0.5 mg/ml, 0.75 mg/ml, 1mg/ml, 2 mg/ml, 3 mg/ml and 4 mg/ml. The obtained results confirm the highly solubilizing action of CMG on resveratrol already starting from the concentration of 0.25 mg/ml, that is with a ratio CMG:resveratrol equal to 1:2 (Figure 4).

However, the solubilization of resveratrol is already evident at the concentration of 0.025 mg/ml, corresponding to a ration CMG:resveratrol of 1:20, and it increases gradually upon increasing the concentration of CMG.

### Example 3 (Figure 5, Figure 6, Figure 7)

Starting from the data of stability of resveratrol in aqueous solution (Figure 5), wherein a strong degradation of the molecule already after 2 months is clear, one proceeded to check the stability of resveratrol in presence of CMG.

The solutions under examination were prepared by using the same aqueous solution described previously and with the following concentrations of resveratrol and CMG:
a) Resveratrol 0.5 mg/ml + CMG 1 mg/ml
b) Resveratrol 1 mg/ml + CMG 1 mg/ml

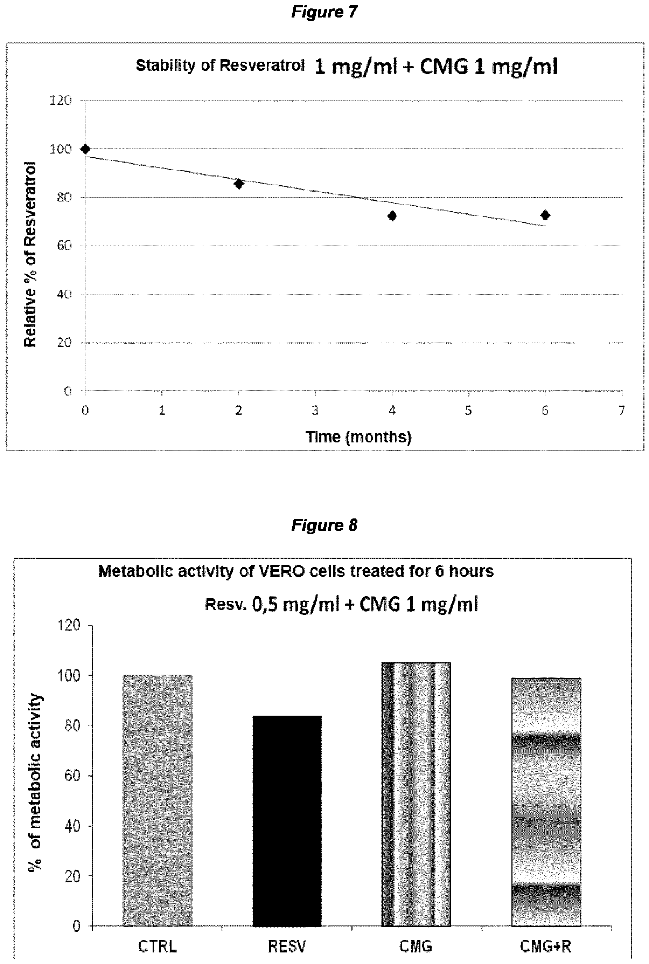

The solutions, kept in closed environment, at controlled temperature (25±2°C) and protected from light, were examined at different times as from the preparation thereof, by obtaining the following results:
- **Solution a)** 11 months after preparation the obtained concentrations of resveratrol were reduced only by 20% with respect to the basal one (Figure 6)
- **Solution b)** 6 months after preparation a reduction of the concentration of resveratrol of about 20% (Figure 7) was detected.

These data show that CMG not only is able to solubilize efficiently resveratrol, but even to stabilize it in time in an aqueous solution, by delaying the natural degradation thereof.

### Example 4 (Figure 8)

Upon considering the application of the association of CMG and resveratrol for the formulation of pharmaceutical products, dietary supplies, medical devices, cosmetics, functional pieces of food, veterinary products and then an interaction with the human biological systems, the safety profile of this association was examined.

In particular tests of metabolic activity on VERO cellular line were performed, by comparing the effect of the single products and the association thereof. The following solutions were used, re-suspended directly in the culture medium:
- Resveratrol 0.5 mg/ml
- CMG 1 mg/ml
- Resveratrol 0.5 mg/ml + CMG 1 mg/ml
The effect of the cellular vitality was evaluated 6 hours after exposition to the products. The so obtained data, compared to those resulting from the not treated cells (CTRL), show that resveratrol on itself, at the concentration of 0.5 mg/ml (2.2 mM), determines 6 hours after exposition 20% of reduction in the cellular metabolic activity, whereas sole CMG has no influence on the cellular vitality. Likewise, the exposition of the cells to the association of resveratrol and CMG at the previously mentioned concentrations (0.5mg/ml of resveratrol + 1 mg/ml of CMG), does not alter in any way the metabolic activity of the cells, showing the surprisingly positive effect exerted by CMG against resveratrol and the natural cytotoxicity thereof (Figure 8). The association object of the present patent thus allows resveratrol to carry out the several biological activities thereof in total safety, by making it even more available at cellular level.

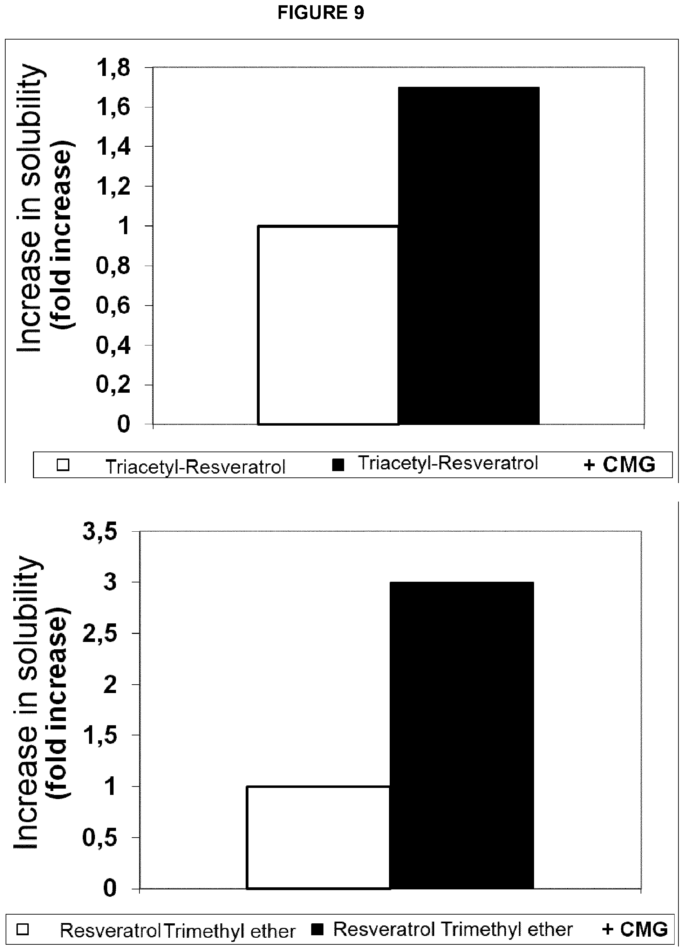

### EXAMPLE 5 (Figure 9)

### Reference

The solubility of triacetyl-resveratrol and of trimethylether of resveratrol in an aqueous solution (of the same composition described in example 1) in presence of CMG was evaluated. In particular, the solubility in aqueous phase of triacetyl-resveratrol and of trimethyl ether of resveratrol was evaluated at first and this value was considered unitary. Then, aqueous solutions of the same substances in presence of 1 mg/mL of CMG were prepared and the solubility thereof was checked, which results to be increased by more than one and a half time for the triacetylresveratrol and by three times for thetrimethylether of resveratrol. The obtained results confirm the highly solubilizing action of CMG on both compounds.

### Bibliography

1. Pietraforte D. 2005. Antiossidanti polifenolici della dieta nella prevenzione delle patologie degenerative. ISS, reports ISTISAN 05/40.
2. Kasdallah-Grissa A, Mornagui B, Aouani E, et al., 2006. Protective effect of resveratrol on ethanol-induced lipid peroxidation in rats. Alcohol Alcoholism 41:236-9.
3. Tsai SH, Lin-Shiau SY, Lin JK. 1999. Suppression of nitric oxide synthase and the down-regulation of the activation of NF-κB in macrophages by resveratrol. British J Pharmacol 126:673-680.
4. Palamara AT, Nencioni L, Aquilano K, et al., 2005. Inhibition of influenza A virus replication by resveratrol. J Infect Dis 191:1719-1729.
5. Drago L, Nicola L, Ossola F, et al., 2008. In vitro antiviral activity of resveratrol against respiratory viruses. J Chemother 20:393-394.
6. Ragione FD, Cucciolla V, Borriello A, et al., 1998. Resveratrol arrests the cell division cycle at S/G2 phase transition. Biochem Biophys Res Commun 250:53-58.
7. Wang LX, Heredia A, Song H, et al., 2004. Resveratrol glucuronides as the metabolites of resveratrol in humans: characterization, synthesis, and anti-HIV activity. J Pharm Sci 93:2448-2457.
8. http://it.wikipedia.org/wiki/Resveratrolo
9. Wang Y, Yu Y, Mao J, 2009. Carboxymethylated beta-glucan derived from Poria cocos with biological activities. J.Agric Food Chem 57 (22):10913-5.
10. Babincovà M, Bacovà Z et al, 2002. Antioxidant properties of carboxymethyl glucan: comparative analysis. J Med Food 5(2): 79-83.
11. Magnani M, Castro-Gomez RJ et al. 2011. Protective effect of carboxymethyl-glucan (CM-G) against DNA damage in patients with advanced prostate cancer. Genet Mol Biol 34 (1):131-5.
12. Schronerovà K, Babincovà M et al, 2007. Carboxymethylated (1-3)-beta-D-glucan protects liposomes against ultraviolet light-induced lipid peroxidation. J Med Food 10 (1): 189-193.
13. Magnani M, Castro-Gomez RH et al, 2012. Analysis of peripheral T cells and the CC chemokine receptor (CCR5) delta 32 polymorphism in prostate cancer patients treated with carboxymethyl-glucan (CM-G). Nat Prod Res,26(10): 945-951.
14. Dergunova MA, Alexeenko TV et al, 2009. Characterization of the novel chemically modified fungal polysaccharides as the macrophage stimulators. Int Immunopharmacol 9(6): 729-33.
15. Majtàn J, Kogan et al, 2005. Stimulation of TNF-alpha release by fungal cell wall polysaccharides. Z Naturforsch C 60 (11-12): 921-6.

## Claims

1. An association of resveratrol and/or salts and carboxymethylglucan, wherein resveratrol and carboxymethylglucan are present in a weight ratio comprised between 20:1 and 1:100.

2. The association according to claim 1 constituted by resveratrol and carboxymethylglucan.

3. The association according to at least one of the claims 1 to 2 for use in a human or veterinary therapeutic treatment.

4. The association according to claim 3 for use in the treatment of inflammatory, viral, diabetic, cardiac, neurodegenerative, and atherosclerotic pathologies.

5. A composition comprising an association according to at least one of the claims 1 to 2 and one or more pharmaceutically/nutraceutically acceptable vehicles and/or diluents and/or excipients and/or additives.

6. The composition according to claim 5 for use in the treatment of inflammatory, viral, diabetic, cardiac, neurodegenerative, and atherosclerotic pathologies.

7. A dietary supply comprising an association as claimed in at least one of the claims 1 to 2.

8. A medical device comprising an association as claimed in at least one of the claims 1 to 2.

9. A functional food comprising an association as claimed in at least one of the claims 1 to 2.

10. A cosmetic product comprising an association as claimed in at least one of the claims 1 to 2.

11. A process for manufacturing the composition according to claim 5, wherein the active mixture comprising resveratrol and/or salts and carboxymethylglucan is formulated into suitable dosage units with one or more pharmaceutically/nutraceutically acceptable excipients and/or additives.

## Patentansprüche

1. Assoziation von Resveratrol und/oder Salzen und Carboxymethylglucan, wobei Resveratrol und Carboxymethylglucan in einem Gewichtsverhältnis, welches in 20:1 bis 1:100 umfasst ist, anwesend sind.

2. Assoziation nach Anspruch 1, die sich aus Resveratrol und Carboxymethylglucan zusammensetzt.

3. Assoziation nach mindestens einem der Ansprüche 1 bis 2 zur Verwendung bei humanmedizinischer oder veterinärmedizinischer therapeutischer Behandlung.

4. Assoziation nach Anspruch 3 zur Verwendung bei der Behandlung von entzündlichen, viralen, diabetischen, kardialen, neurodegenerativen und atherosclerotischen Pathologien.

5. Zusammensetzung, die eine Assoziation nach mindestens einem der Ansprüche 1 bis 2 und ein oder mehrere pharmazeutisch/nutrazeutisch verträgliche Vehikel und/oder Verdünnungsmittel und/oder Exzipienten und/oder Zusatzstoffe umfasst.

6. Zusammensetzung nach Anspruch 5 für die Verwendung bei der Behandlung von entzündlichen, viralen, diabetischen, kardialen, neurodegenerativen und atherosclerotischen Pathologien.

7. Diätetisches Mittel, das eine wie in mindestens einem von Ansprüchen 1 bis 2 beanspruchte Assoziation umfasst.

8. Medizinprodukt, das eine wie in mindestens einem von Ansprüchen 1 bis 2 beanspruchte Assoziation umfasst.

9. Funktionelles Lebensmittel, das eine wie in mindestens einem von Ansprüchen 1 bis 2 beanspruchte Assoziation umfasst.

10. Kosmetisches Produkt, das eine wie in mindestens einem von Ansprüchen 1 bis 2 beanspruchte Assoziation umfasst.

11. Verfahren zur Herstellung der Zusammensetzung nach Anspruch 5, wobei das aktive Gemisch umfassend Resveratrol und/oder Salze und Carboxymethylglucan, in geeigneten Dosierungseinheiten mit einem oder mehreren pharmazeutisch/nutrazeutisch verträglichen Exzipienten und/oder Zusatzstoffen formuliert wird.

## Revendications

1. Association de resvératrol et/ou de ses sels et de carboxyméthylglucane, dans laquelle le resvératrol et le carboxyméthylglucane sont présents dans un rapport pondéral situé entre 20/1 et 1/100.

2. Association selon la revendication 1 constituée de resvératrol et de carboxyméthylglucane.

3. Association selon au moins l'une des revendications 1 à 2 pour une utilisation dans un traitement thérapeutique humain ou vétérinaire.

4. Association selon la revendication 3 pour une utilisation dans le traitement de pathologies inflammatoires, virales, diabétiques, cardiaques, neurodégénératives, et athéroscléreuses.

5. Composition comprenant une association selon au moins l'une des revendications 1 à 2 et un ou plusieurs véhicules et/ou diluants et/ou excipients et/ou additifs acceptables pour un usage pharmaceutique/nutraceutique.

6. Composition selon la revendication 5 pour une utilisation dans le traitement de pathologies inflammatoires, virales, diabétiques, cardiaques, neurodégénératives, et athéroscléreuses.

7. Apport alimentaire comprenant une association telle que revendiquée dans au moins l'une des revendications 1 à 2.

8. Dispositif médical comprenant une association telle que revendiquée dans au moins l'une des revendications 1 à 2.

9. Aliment fonctionnel comprenant une association telle que revendiquée dans au moins l'une des revendications 1 à 2.

10. Produit cosmétique comprenant une association telle que revendiquée dans au moins l'une des revendications 1 à 2.

11. Procédé de fabrication de la composition selon la revendication 5, dans lequel le mélange actif comprenant du resvératrol et/ou ses sels et du carboxyméthylglucane est formulé dans des unités posologiques appropriées avec un ou plusieurs excipients et/ou additifs acceptables pour un usage pharmaceutique/nutraceutique.
